# EUROPEAN PATENT APPLICATION

(11) **EP 4 459 633 A1**
(43) Date of publication of application: **06.11.2024**
(21) Application number: 23170914.8
(22) Date of filing: 01.05.2023
(51) Int. Cl.: G16H 40/67, G06Q 10/0631, G06Q 50/22, G16H 40/20, G16H 50/70

(54) **SYSTEMS AND METHODS OF PRIORITIZING INTERVENTION IN REMOTE PATIENT MONITORING PROGRAMS TO IMPROVE PATIENT OUTCOMES**

(71) Applicant: Koninklijke Philips N.V., 5656 AG Eindhoven (NL)
(72) Inventor: VAN DE CRAEN, Dieter Maria Alfons, 5656 AG Eindhoven (NL); PIJL, Marten Jeroen, 5656 AG Eindhoven (NL); SOKORELI, Ioanna, 5656AG Eindhoven (NL); RICCI LOPES, Ricardo, 5656 AG Eindhoven (NL)
(74) Representative: Philips Intellectual Property & Standards

(57) **Abstract**

Provided herein are systems and methods of providing engagement guidance to virtual care teams in connection with a plurality of patients that are enrolled in a remote patient monitoring program. These systems and methods may be provided as a standalone service or as an add-on to a patient data management system (PDMS) and/or a patient monitoring service. These systems and methods may find particular use in connection with remote patient monitoring environments where each virtual care team or virtual care coach is responsible for a large number of patients (e.g., tens to hundreds of patients).

## Description

### FIELD OF THE INVENTION

The present disclosure relates generally to remote patient monitoring systems and methods of remotely monitoring patients, and, more specifically, to systems and methods for providing patient engagement guidance and prioritizing intervention in remote patient monitoring (RPM) programs in order to improve patient outcomes.

### BACKGROUND OF THE INVENTION

Adoption of remote patient monitoring (RPM) programs has accelerated since the COVID-19 pandemic and has been elevated from a "nice-to-have" option to a vital component of patient care in many scenarios, especially in the case of chronic care management. These remote patient monitoring programs provide care teams with the tools they need to remotely track the health status of their patients at home, collaborate with the patients' physicians, and help detect health issues before they lead to hospital admission or readmission for the patients in such programs.

Many factors are driving the growth of the remote patient monitoring market. For example, the rising geriatric population and the growing need to expand healthcare access, cost benefits of telehealth and RPM, benefits of RPM to reduce the burden on medical resources, advancements in telecommunications, growing incidences of chronic diseases, and increasing investments in telehealth and RPM are some of the major factors driving this growth. Further, advances in monitoring hubs, smartphone platforms, sensor technologies, cellular networks, cloud computing, and mobile and wearable medical devices have removed many of the technical barriers to RPM. Although some key barriers to the adoption of RPM solution are now being removed, many challenges remain.

### SUMMARY OF THE INVENTION

Under a remote patient monitoring program, providers regularly assign patients to specific care protocols and interventions that are tailored to the relevant medical condition or acuity level, including measuring and submitting vital sign data, test results, completing surveys, watching educational videos, and participating in telehealth appointments with the virtual care team, among other monitoring-related activities. In a typical RPM solution, virtual care team or virtual care coach often manage a large population consisting of hundreds of patients. Thus, one of the key challenges in a remote patient monitoring program is keeping patients engaged while optimizing the use of available resources. According to the present disclosure, it is recognized that in order to optimally impact the outcomes of such a patient population, the virtual care team or virtual care coach will need to reach out to the right patients, in the right manner, at the right times, and with the right content. As such, provided herein are technical solutions that provide clear guidance on the prioritization of patients under remote monitoring and facilitate data-driven intervention actions that drive the biggest impact on patient outcomes.

According to an embodiment of the present disclosure, a remote patient monitoring system configured to provide engagement guidance in connection with a plurality of patients is provided. The system can include: one or more processors; and memory having stored thereon machine-readable instructions that, when executed by the one or more processors, cause the one or more processors to perform operations comprising: (i) obtain patient data for at least a first subset of patients from one or more data sources; (ii) extract a plurality of experience level features for each patient of at least the first subset of patients from the patient data obtained; (iii) determine a condition experience level for each patient of at least the first subset of patients based on the experience level features extracted for the corresponding patient; (iv) generate an intervention priority score for each patient of at least the first subset of patients by applying a trained intervention priority model to the condition experience level determined for the corresponding patient, wherein each intervention priority score is indicative of a likelihood of the corresponding patient having a positive change in one or more clinical outcomes following one or more intervention actions; and (v) automatically generate a recommended intervention plan for each patient of at least the first subset of patients based on the intervention priority scores generated for at least the first subset of patients, wherein each recommended intervention plan includes at least one intervention action.

In an aspect, the intervention priority model may be trained by: (i) obtaining historical patient data for a plurality of historical patients, wherein the historical patient data includes historical intervention action data and actual patient outcome data for the plurality of historical patients; (ii) extracting a plurality of experience level features for each historical patient from the historical patient data obtained; (iii) determine a condition experience level for each historical patient based on the experience level features extracted for the corresponding historical patient; (iv) training the intervention priority model using the condition experience levels determined for each of the historical patients and the historical intervention action data for each of the historical patients as inputs and changes in actual patient outcome data for each of the historical patients as outputs; and (v) storing the trained intervention priority model.

In an aspect, remote patient monitoring system can further include: one or more patient interfaces configured to be executed by or otherwise accessible via one or more patient devices, each patient interface being configured to send and receive data related to the remote monitoring of a corresponding patient; and one or more coach interfaces configured to be executed by or otherwise accessible via one or more coach devices, each coach interface being configured to send and receive data related to the remote monitoring of a plurality of patients.

In an aspect, each recommended intervention plan can include at least one intervention action identified based on the intervention priority score generated for the corresponding patient.

In an aspect, the patient data can include one or more of the following: demographic information; patient-reported outcome measures; patient-reported experience measures; user interface usage data; system usage data; clinical data; socio-economic data; and historical intervention action data.

In an aspect, the intervention priority score generated for each patient can include a plurality of sub-scores corresponding to one or a combination of potential intervention actions, wherein each sub-score is indicative of a likelihood of the corresponding patient having a positive change in one or more clinical outcomes following the one or the combination of potential intervention actions.

In an aspect, the condition experience level determined for each patient can have a first component comprising a score based on one or more current clinical values associated with the corresponding patient, and a second component comprising a score based on evidence of one or more historical intervention actions.

According to another aspect of the present disclosure, a non-transitory computer-readable storage medium having stored thereon machine-readable instructions is provided. When executed by one or more processors, the machine-readable instructions stored thereon may cause the one or more processors to perform operations including: (i) obtaining patient data for at least a first subset of patients from one or more data sources; (ii) extracting a plurality of experience level features for each patient of at least the first subset of patients from the patient data obtained; (iii) determining a condition experience level for each patient of at least the first subset of patients based on the experience level features extracted for the corresponding patient; (iv) generating an intervention priority score for each patient of at least the first subset of patients by applying a trained intervention priority model to the condition experience level determined for the corresponding patient, wherein each intervention priority score is indicative of a likelihood of the corresponding patient having a positive change in one or more clinical outcomes following one or more intervention actions; and (v) automatically generating a recommended intervention plan for each patient of at least the first subset of patients based on the intervention priority scores generated for at least the first subset of patients, wherein each recommended intervention plan includes at least one intervention action.

In an aspect, the intervention priority model may be trained by: (i) obtaining historical patient data for a plurality of historical patients, wherein the historical patient data includes historical intervention action data and actual patient outcome data for the plurality of historical patients; (ii) extracting a plurality of experience level features for each historical patient from the historical patient data obtained; (iii) determine a condition experience level for each historical patient based on the experience level features extracted for the corresponding historical patient; (iv) training the intervention priority model using the condition experience levels determined for each of the historical patients and the historical intervention action data for each of the historical patients as inputs and changes in actual patient outcome data for each of the historical patients as outputs; and (v) storing the trained intervention priority model.

According to another embodiment of the present disclosure, a computer-implemented method of providing engagement guidance in connection with a plurality of patients using a remote patient monitoring system is provided. The method includes: obtaining patient data for at least a first subset of patients from one or more data sources; extracting a plurality of experience level features for each patient of at least the first subset of patients from the patient data obtained; determining a condition experience level for each patient of at least the first subset of patients based on the experience level features extracted for the corresponding patient; generating an intervention priority score for each patient of at least the first subset of patients by applying a trained intervention priority model to the condition experience level determined for the corresponding patient, wherein each intervention priority score is indicative of a likelihood of the corresponding patient having a positive change in one or more clinical outcomes following one or more intervention actions; and automatically generating a recommended intervention plan for each patient of at least the first subset of patients based on the intervention priority scores generated for at least the first subset of patients, wherein each recommended intervention plan includes at least one intervention action.

In an aspect, the intervention priority model may be trained by: (i) obtaining historical patient data for a plurality of historical patients, wherein the historical patient data includes historical intervention action data and actual patient outcome data for the plurality of historical patients; (ii) extracting a plurality of experience level features for each historical patient from the historical patient data obtained; (iii) determine a condition experience level for each historical patient based on the experience level features extracted for the corresponding historical patient; (iv) training the intervention priority model using the condition experience levels determined for each of the historical patients and the historical intervention action data for each of the historical patients as inputs and changes in actual patient outcome data for each of the historical patients as outputs; and (v) storing the trained intervention priority model.

In an aspect, the method can further include: presenting, via one or more coach interfaces of the remote patient monitoring system, the recommended intervention plans for at least some of the first subset of patients; receiving, via the one or more coach interfaces of the remote patient monitoring system, a selection of one or more intervention actions from the recommended intervention plans of at least one patient; and automatically implementing, via one or more patient interfaces, at least one intervention action of the one or more selected intervention actions, wherein the at least one intervention action is implemented in connection with at least a first patient.

In an aspect, the method can further include: determining a change in one or more patient outcomes for at least the first patient following the implementation of at least the one intervention action; and updating the trained intervention priority model using an updated training dataset that includes the at least one intervention action and the change in one or more patient outcomes for at least the first patient.

In an aspect, generating an intervention priority score for each patient of at least the first subset of patients may include generating a plurality of sub-scores for each patient of at least the first subset of patients by applying the trained intervention priority model, wherein each sub-score is indicative of a likelihood of the corresponding patient having a positive change in one or more clinical outcomes following one or combination of potential intervention actions.

In an aspect, the patient data can include one or more of the following: demographic information; patient-reported outcome measures; patient-reported experience measures; user interface usage data; system usage data; clinical data; socio-economic data; and historical intervention action data.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a diagram illustrating a remote patient monitoring environment covering multiple patient populations according to aspects of the present disclosure.
FIG. 2A is a flowchart illustrating a computer-implemented method of providing engagement guidance in connection with a plurality of patients using a remote patient monitoring system according to aspects of the present disclosure.
FIG. 2B is a flowchart illustrating a computer-implemented method of providing engagement guidance in connection with a plurality of patients using a remote patient monitoring system according to additional aspects of the present disclosure.
FIG. 3 is a diagram illustrating the architecture for remote patient monitoring system according to aspects of the present disclosure.
FIG. 4 is a diagram illustrating different components of a remote patient monitoring system according to aspects of the present disclosure.
FIG. 5 is a flowchart illustrating a computer-implemented process for training an intervention priority prediction model according to aspects of the present disclosure.

### DETAILED DESCRIPTION OF EMBODIMENTS

Provided herein are systems and methods of providing engagement guidance to virtual care teams in connection with a plurality of patients that are enrolled in a remote patient monitoring program. These systems and methods may be provided as a standalone service or as an add-on to a patient data management system (PDMS) and/or a patient monitoring service. These systems and methods may find particular use in connection with remote patient monitoring environments where each virtual care team or virtual care coach is responsible for a large number of patients (e.g., tens to hundreds of patients).

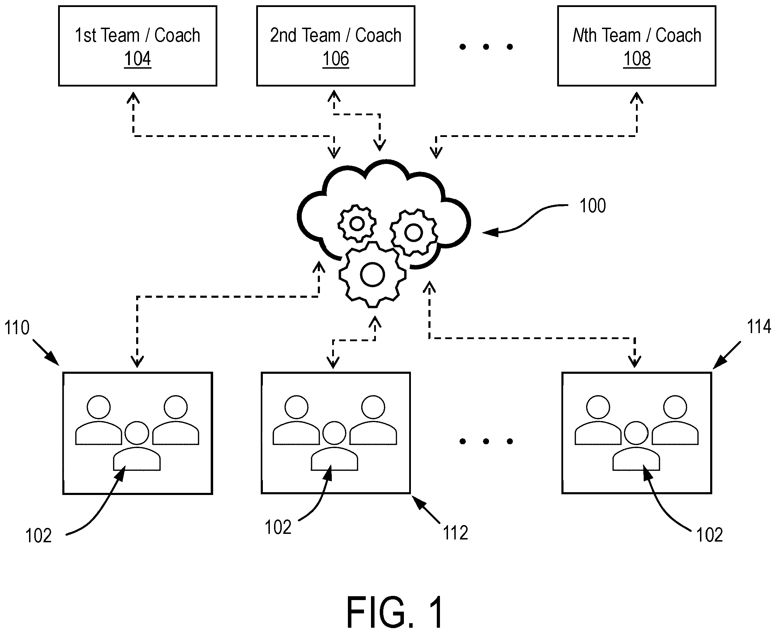

For example, with reference to FIG. 1, a remote patient monitoring system 100 configured to provide engagement guidance in connection with a plurality of patients 102 is illustrated in accordance with various aspects of the present disclosure. The remote patient monitoring system 100 may be provided as a web-based and/or cloud-based service, which may be a component of a patient data management system (PDMS). The remote patient monitoring system 100 may be utilized by a number of virtual care teams or virtual care coaches 104, 106, 108 in order to improve patient outcomes by providing data-driven intervention guidance that keeps patients 102 engaged in their respective remote patient monitoring programs.

As described herein, the remote patient monitoring system 100 can be configured to provide engagement guidance in connection with a plurality of patients 102, including one or more subsets 110, 112, 114 of patients 102. For example, the remote patient monitoring system 100 may be utilized by multiple virtual care teams 104, 106, 108 that are each responsible for a different subset 110, 112, 114 of patients 102. In embodiments, each subset or group 110, 112, 114 of patients 102 that a virtual care team 104, 106, 108 is responsible may vary, including from tens to hundreds of patients 102. Further, any number of virtual care teams 104, 106, 108 and any number of patients 102 may utilize the remote patient monitoring system 100. Put another way, the remote patient monitoring system 100 of the present disclosure may be scaled to cover large networks of patients 102 and virtual care teams 104, 106, 108.

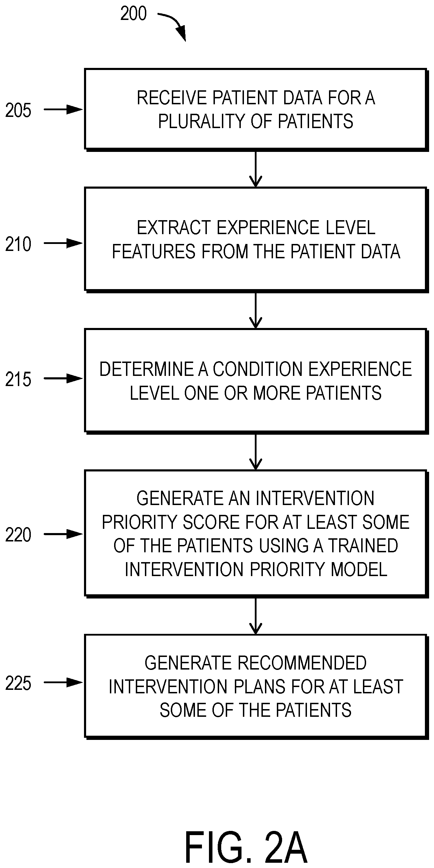

The remote patient monitoring system 100 may be configured to perform one or more steps of the methods and/or processes described herein. For example, with reference to FIG. 2A, a method 200 of providing engagement guidance in connection with a plurality of patients (e.g., patients 102) using a remote patient monitoring system (e.g., system 100) is illustrated according to various aspects of the present disclosure. As shown, the method 200 can include: in a step 205, obtaining patient data for at least a first subset of patients from one or more data sources; in a step 210, extracting a plurality of experience level features for each patient of at least the first subset of patients from the patient data obtained; in a step 215, determining a condition experience level for each patient of at least the first subset of patients based on the experience level features extracted for the corresponding patient; in a step 220, generating an intervention priority score for each patient of at least the first subset of patients by applying a trained intervention priority model to the condition experience level determined for the corresponding patient, wherein each intervention priority score is indicative of a likelihood of the corresponding patient having a positive change in one or more clinical outcomes following one or more intervention actions; and in a step 225, automatically generating a recommended intervention plan for each patient of at least the first subset of patients based on the intervention priority scores generated, wherein each recommended intervention plan includes at least one intervention action.

At the step 205, the computer-implemented method 200 includes obtaining patient data for at least a first subset of patients from one or more data sources. In embodiments, the first subset of patients can be a first group 110, 112, 114 of patients 102 that are associated with a particular virtual care team 104, 106, 108. Put another way, the step 205 includes obtaining patient data for at least a first group 110, 112, 114 of patients 102 that have a common virtual care team or coach 104, 106, 108.

In embodiments, the patient data can include one or more of the following types of information for each of a plurality of patients: demographic information, such as age, gender, sex, race, ethnicity, marital status, and/or the like; patient-reported outcome measures (PROMS), including general and condition-specific PROMS; patient-reported experience measures (PREMS), including general and condition-specific PREMS; system usage data, such as logins, time active, activities performed, devices used, and/or the like; clinical data, such as various at-home test results and/or lab test results; socio-economic data, including education level, zip code, urbanity, and/or the like; and historical intervention action data, including records of past intervention actions and/or educational materials provided. A more detailed list of the types of patient data contemplated by the present disclosure is shown in Table 1 below:

**TABLE 1. EXAMPLES OF PATENT DATA**

| **Variable** | **Type** | **Values** |
|---|---|---|
| Age | Demographic | >= 0 (days, months, years) |
| Gender | Demographic | M,F,NB,N/A |
| Sex | Demographic | M,F,IS |
| Race / Ethnicity | Demographic | E.g., American Indian or Alaska Native, Asian, Black or African American, Hispanic or Latino, Native Hawaiian or Other Pacific Islander... |
| Marital Status | Demographic | Single, Married |
| Blood Glucose | Clinical | mg/dL or mmol/L |
| HbA1C | Clinical | % |
| Comorbidities | Clinical | Heart Failure, Hypertension, COPD, Obesity, Limited mobility |
| Smoking status | Clinical | Yes/No |
| Complications | Clinical | Retinopathy, neuropathy (e.g., for diabetes patients) |
| Logins | Solution usage / behavior | # of attempts, # of successful or unsuccessful logins |
| Time active | Solution usage / behavior | Duration (seconds, minutes, hours, etc.) |
| Measurements taken | Solution usage / behavior | Type of measurement and/or # of times performed |
| Medication refills | Solution usage / behavior | # of refills and timing of refills per medication |
| Technical challenges | Solution usage / behavior | Reports or requests for technical assistance |
| Devices used | Solution usage / behavior | Glucose monitor, connected weight scale, blood pressure monitor |
| Education | Socio-economic | High-school degree, college degree, professional degree, etc. |
| Estimated income (zip code) | Socio-economic | Zip code-based economic information |
| Index of social advantage / disadvantage | Socio-economic | Decile of relative social disadvantage |
| Urbanity | Socio-economic | Rural, urban |
| General PROMS | PROMS & PREMS | World Health Organisation- Five Well-Being Index (WHO-5) |
| General PROMS | PROMS & PREMS | Euroqol-5D (EQ-5D) |
| Condition-specific PROMS | PROMS & PREMS | Audit of Diabetes Dependent Quality of Life (ADDQL), |
| Condition-specific PROMS | PROMS & PREMS | Summary of Diabetes Self-Care Activities Measure (SDSCA) |
| Condition-specific PREMS | PROMS & PREMS | Patient Experience of Diabetes Services Survey |
| Coaching calls | Interventions | Information shared, resolving (technical) obstacles |
| Distribution of educational materials | Interventions | Instructions on device usage, dietary recommendations |

It should be appreciated that this list is non-exhaustive and that the patient data can include other types of general and/or condition-specific information. For example, in some embodiments, the patient-reported experience and outcome measures for people living with diabetes can include those described in "Patient-Reported Experience and Outcome Measures in People Living with Diabetes: A Scoping Review of Instruments," PATIENT 14, 759-773 (2021) https://doi.org/10.1007/s40271-Q21-00526-v, the contents of which are herein incorporated by reference in their entirety.

The patient data may be obtained from one or more different data sources. For example, at least some of the information may be collected via publicly accessible sources and/or records, while other information may be collected based on self-reported means (e.g., surveys, in-take forms, etc.). In embodiments, at least some of the patient data can be collected from one or more an electronic medical records databases. In further embodiments, at least some of the patient data may be stored in one or more databases of the remote patient monitoring system (e.g., remote patient monitoring system 100) itself, including but not limited to, solution usage and user behavior, coaching interventions and outcomes, and/or the like.

At the step 210, the computer-implemented method 200 includes extracting a plurality of experience level features for each patient of the first subset of patients from the patient data that is obtained. That is, the step 210 includes extracting experience level features from the patient data for each patient 102 of at least one of the patient groups 110, 112, 114. As described herein, the term "experience level features" refer to one or a combination of factors, results, or trends in the patient data that relate to how experienced a particular patient 102 may be with having and/or managing a particular medical condition (e.g., diabetes, etc.).

In some embodiments, one or more of the experience level features may include results and/or trends of clinical parameters that are associated with a particular medical condition. For example, in a patient 102 living with diabetes, current and/or historical values and trends for the patient's blood glucose and HbA1C may be calculated or otherwise extracted as experience level features. In other embodiments, one or more of the experience level features may include evidence or records of historical intervention actions taken with respect to a particular patient 102. For example, if a patient 102 has received 10 coaching calls over the past 3 months, those parameters may be extracted or otherwise quantified as experience level features.

At the step 215, the computer-implemented method 200 includes determining a condition experience level for each patient of at least the first subset of patients based on the experience level features extracted for the corresponding patient. That is, the step 215 includes determining a condition experience level for each patient 102 of at least one of the patient groups 110, 112, 114 based on the extracted experience level features for the corresponding patient 102.

In embodiments, the condition experience level is a condition-specific metric that is determined for each patient 102 and is a composite of two components: (1) a first component comprising a score based on one or more current clinical values associated with the patient 102; and (2) a second component comprising a score based on evidence of one or more historical intervention actions. As mentioned above, the first component gives an indication of how well a patient 102 is managed with the underlying idea that if a patient 102 is already well managed, then the patient's priority will be lower. Similarly, the second component encompasses past coaching and educational material usage, which is translated into a score that gives an indication of the amount of condition-specific knowledge the patient 102 has been exposed to. In embodiments, interactions between the two components of the condition experience level for a particular patient 102 may be handled by looking at the change in the patient's clinical outcomes in the period immediately after an intervention action.

At the step 220, the computer-implemented method 200 includes generating an intervention priority score for each patient of at least the first subset of patients by applying a trained intervention priority model to the condition experience level determined for the corresponding patient. As described herein, an intervention priority score is indicative of a likelihood of the corresponding patient 102 having a positive change in one or more clinical outcomes following one or more potential intervention actions.

In particular embodiments, generating an intervention priority score for a particular patient 102 includes generating a plurality of sub-scores for the patient 102, wherein each sub-score corresponds to one or a combination of potential intervention actions. Put another way, the intervention priority score for a particular patient 102 can include a plurality of sub-scores for the patient 102, wherein each sub-score is indicative of a likelihood that a particular intervention action or combination of intervention actions will have a positive change on one or more clinical outcomes of the patient 102. For example, if there are five potential intervention actions that a virtual care team may take, then the intervention priority score may include sub-scores for each of those five intervention actions and sub-scores any combination of intervention actions thereof.

At the step 225, the computer-implemented method 200 includes automatically generating a recommended intervention plan for one or more patients based on the intervention priority scores generated for those patients. This step may include generating recommended intervention plans for each of the patients 102 of the subset 110, 112, 114 of patients, or may include generating recommended intervention plans for some of those patients 102. In particular embodiments, the recommended intervention plans may be ranked based on the generated intervention priority scores such that the recommended intervention plans comprises a list of patients ordered according to their impactability.

In particular embodiments, selection of the appropriate recommended intervention actions may be performed in a number of potential ways. For example, based on a particular patient's profile, the patient can be classified into a group of similar patients using a clustering approach, and the most appropriate intervention action may be recommended based on the intervention priority score and the characteristics of the group or cluster. More specifically, it may be known that a particular group or cluster of less technology-savvy patients would benefit from a human-to-human interaction, and therefore the recommendation for a patient in that group may include that type of intervention action. It should be appreciated that approaches other than clustering may be used to determine groups of similar patient profiles.

Each recommended intervention plan can include at least one recommended intervention action that the virtual care team 104, 106, 108 may take with respect to a corresponding patient 102. For example, the recommended intervention actions can include one or more of the following: a health coach call; a physician interaction; a customer support call; an email; a text message; a device message; a sharing of educational content; an interactive voice response; a survey; and continue to monitor (i.e., no action taken). More specifically, the recommended interventions can include a recommendation that the virtual care team or virtual coach call a patient, a recommendation that the patient's treating physician follow-up with the patient, a recommendation that customer support call the patient, a recommendation that an automated email or email from the virtual care team be sent to the patient, a recommendation that an automated text message or text message from the virtual care team be sent to the patient, a recommendation that an automated message be sent to the patient via a patient device, a recommendation that certain educational content be shared with the patient, a recommendation that an interactive voice response to shared with the patient, a recommendation that a survey be sent to the patient, and/or a recommendation to continue monitoring the patient without further action, including combinations thereof.

In particular embodiments, the recommended intervention plans generated can include one or more intervention actions that have an intervention priority score (or an intervention priority sub-score) above a predefined threshold. That is, if a particular intervention action or combination of intervention actions only has an intervention priority score (or sub-score) of less than 50%, then that intervention action or combination of intervention actions may be excluded from the recommended intervention plan while other intervention actions or combinations of intervention actions having a priority score (or sub-score) of greater than 50% may be included. It should be appreciated that a 50% threshold is only provided as an example and other thresholds are contemplated. In particular embodiments, this threshold may be predefined for the system or tailored based on the intervention priority scores being generated.

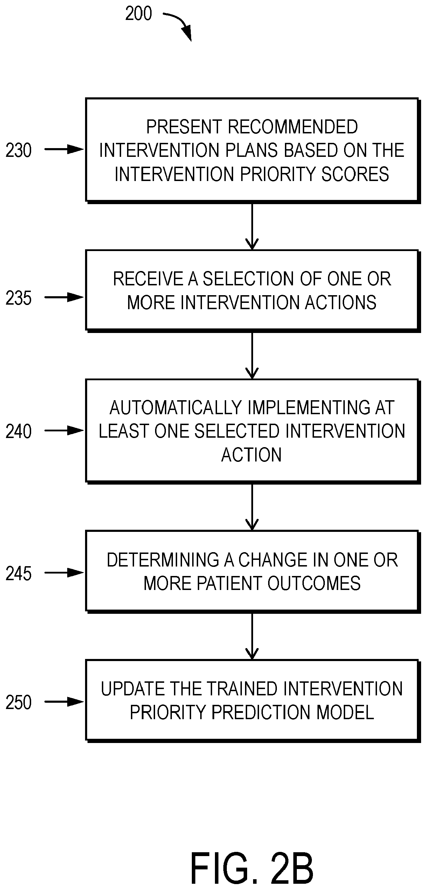

With reference to FIG. 2B, the computer-implemented method 200 may further include one or more of the following: in a step 230, presenting the recommended intervention plans for at least some of the first subset of patients via one or more coach interfaces of the remote patient monitoring system; in a step 235, receiving a selection of one or more intervention actions from the recommended intervention plan of at least one patient via the one or more coach interfaces; in a step 240, automatically implementing at least one of the selected intervention actions via one or more patient interfaces, wherein the intervention action is implemented in connection with at least a first patient; in a step 245, determining a change in one or more patient outcomes for at least the first patient following the implementation of at least the one intervention action; and in a step 250, updating the trained intervention priority prediction model using an updated training dataset that includes the implemented intervention actions and the change in the one or more patient outcomes for at least the first patient.

At the step 230, the computer-implemented method 200 includes presenting the recommended intervention plans for at least some of the first subset of patients 102 (e.g., one of groups 110, 112, 114). The recommended intervention plans may be presented to a virtual care team or virtual care coach 104, 106, 108 via one or more coach interfaces of the remote patient monitoring system 100. In embodiments, the recommended intervention plans may be presented as an ordered list of patients 102 according to their impactability (i.e., their generated intervention priority scores). In some embodiments, only those patients 102 having an intervention priority score above a certain threshold (as described above) may be presented to the virtual care team or coach 104, 106, 108.

At the step 235, the computer-implemented method 200 includes receiving a selection of one or more intervention actions from the virtual care team 104, 106, 108 via the one or more coach interfaces. In embodiments, the selections made by the virtual care teams 104, 106, 108 can include one or more intervention actions selected from the recommended intervention plans presented to the virtual care teams 104, 106, 108.

At the step 240, the computer-implemented method 200 includes automatically implementing at least one intervention action of the selected intervention actions. In embodiments, one or more of the selected intervention actions may be implemented via a patient interface associated with a patient 102. In some embodiments, implementing a particular intervention action may include generating instructions for the intervention action to occur.

More specifically, implementing an intervention action can include scheduling and/or conducting a virtual care team or virtual coach call with a patient, scheduling and/or conducting a follow-up interaction between the patient and the patient's treating physician, scheduling and/or conducting a customer support call with the patient, sending an auto-generated email or generating instructions for the virtual care team to send an email to the patient, sending an auto-generated text message or generating instructions for the virtual care team to send a text message to the patient, sending an auto-generated message via a patient device associated with a patient, automatically sharing certain educational content with the patient, automatically generating and sending an interactive voice response to the patient, automatically generating and sending a survey to the patient, and/or continuing to monitor the patient without further action, including combinations thereof.

At the step 245, the computer-implemented method 200 includes determining a change in one or more patient outcomes for at least a first patient for whom an intervention action was implemented. Put another way, the step 245 includes determining whether there were any changes in any measured patient outcomes for any number of patients that received an intervention action in accordance with the present disclosure. For example, the change in patient outcomes can include changes in patient-reported outcome and experience measures, changes in compliance fraction (e.g., fraction of days with glucose readings out of the last 30 days, etc.), changes in testing frequency, changes in clinical test results, and/or the like.

At the step 250, the computer-implemented method 200 includes updating the trained intervention priority prediction model based on the intervention actions that were implemented in the step 240 and the changes in patient outcomes observed in the step 245. In embodiments, the intervention priority prediction model may be retrained based on an updated training dataset that includes at least one of the implemented intervention actions for at least a first patient and the changes in one or more patient outcomes for at least the first patient. In further embodiments, the updated training dataset can include a plurality of intervention actions that were newly implemented for a plurality of patients and any changes in patient outcomes for the corresponding patients.

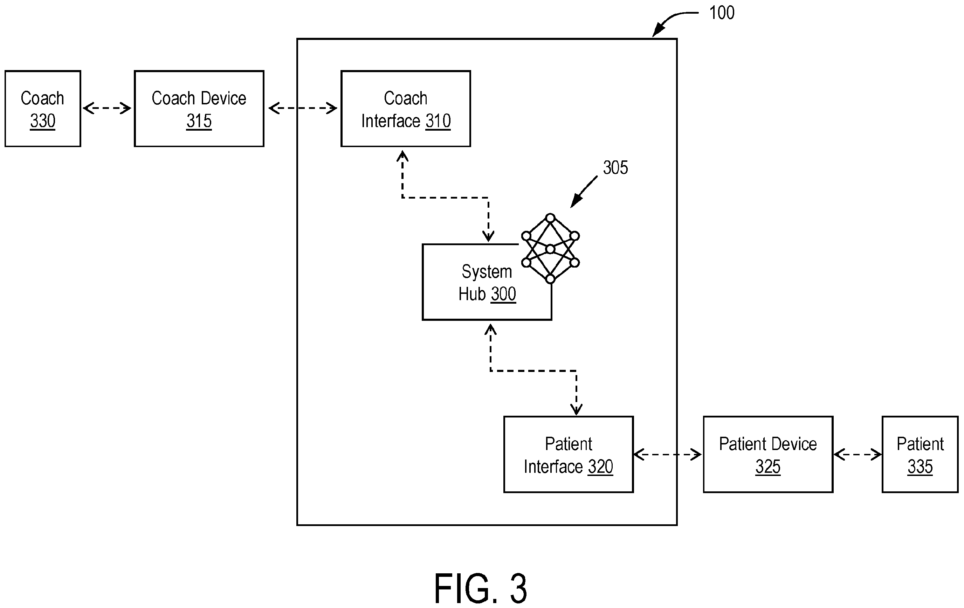

With reference to FIG. 3, a functional block diagram of a remote patient monitoring system 100 adapted to perform the methods and/or processes described herein is illustrated in accordance with further aspects of the present disclosure. In particular, the remote patient monitoring system 100 can include: a system hub 300 configured to perform one or more steps of the methods and/or processes described herein; a trained intervention priority prediction model 305 configured to predict a likelihood of a patient having a positive change in one or more clinical outcomes following one or more intervention actions based on at least a condition experience level for the patient; one or more virtual care team / coach interfaces 310 configured to be executed by or otherwise accessible via one or more coach devices 315; and one or more patient interfaces 325 configured to be executed by or otherwise accessible via one or more patient devices 325.

In the example of FIG. 3, a virtual care team or coach 330 may be able to interact with an associated patient 335 through coach interface 310 of the system 100, including by receiving one or more recommended intervention plans and implementing one or more recommended intervention actions. Similarly, the patient 335 may be able to interact with the virtual care team or coach 330 via a patient interface 320 of the system 100, including by participating in one or more intervention actions (e.g., receiving educational materials, conducting physician or care team appointments, etc.). In embodiments, the coach interface 310 can be an application that is stored on a coach device 315 or can be a web-based service accessible via, for example, a uniform resource locator (URL) using the coach device 315. In some embodiments, the coach device 315 can be a desktop computer or a mobile electronic device, including but not limited to a tablet, a smartphone, a laptop, and/or the like. Similarly, the patient interface 320 can be an application that is stored on a patient device 325 or can be a web-based service accessible via, for example, another URL using the patient device 325. In embodiments, the patient device 325 can be a desktop computer or a mobile electronic device, including but not limited to a tablet, a smartphone, a laptop, and/or the like.

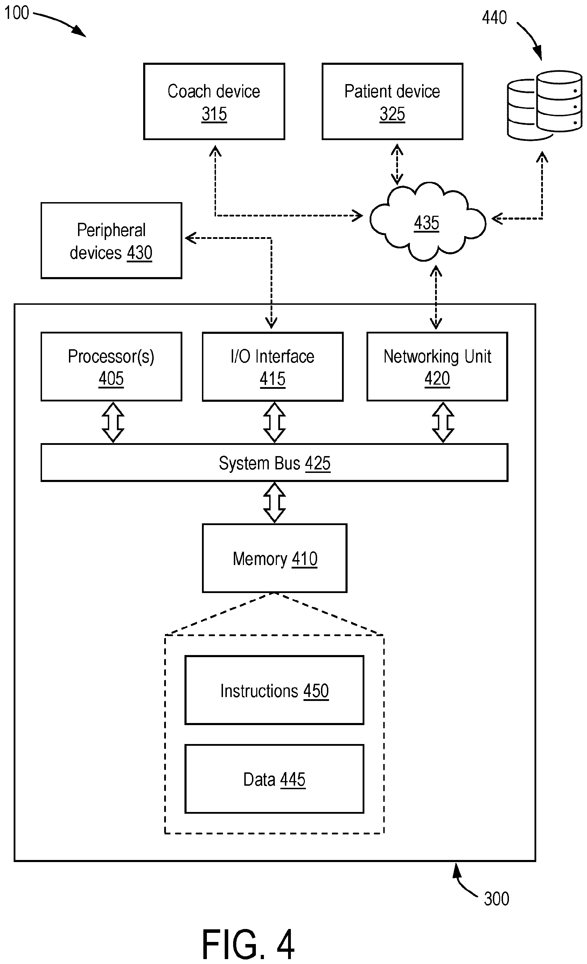

With reference to FIG. 4, a block diagram illustrating a remote patient monitoring system 100 is illustrated according to still further aspects of the present disclosure. As shown, the remote patient monitoring system 100 can include a system hub 300, which can be at least part of a larger patient data management system (PDMS). In embodiments, the remote patient monitoring system 100 includes one or more processors 405, machine-readable memory 410, an input/output interface 415, and/or a networking unit 420, all of which may be interconnected and/or communicate through a system bus 425 containing conductive circuit pathways through which instructions (e.g., machine-readable signals) may travel to effectuate communication, tasks, storage, and the like.

The one or more processors 405 may include one or more high-speed data processors adequate to execute the program components described herein and/or perform one or more steps of the methods described herein. The one or more processors 405 may include a microprocessor, a multi-core processor, a multithreaded processor, an ultra-low voltage processor, an embedded processor, and/or the like, including combinations thereof. The one or more processors 405 may include multiple processor cores on a single die and/or may be a part of a system on a chip (SoC) in which the processor 405 and other components are formed into a single integrated circuit, or a single package. As a non-exhaustive list, the one or more processors 405 can include one or more of an Intel^{®} Architecture Core^{™} based processor, such as a Quark^{™}, an Atom^{™}, an i3, an i5, and i7, or an MCU-class processor, an Advanced Micro Devices, Inc. (AMD) processor such as a Ryzen or Epyc based processor, an A5-A10 processor from Apple^{®} Inc., a Snapdragon^{™} processor from Qualcomm^{®} Technologies, Inc., and/or the like.

The input/output (I/O) interface 415 of the system 100 may include one or more I/O ports that provide a physical connection to one or more devices, such as one or more peripheral devices 430. Put another way, the I/O interface 415 may be configured to connect one or more peripheral devices of the system 100 in order to facilitate communication and/or control of between such devices. In some embodiments, the I/O interface 415 can comprise one or more serial ports.

The networking unit 420 of the recovery prediction system 100 may include one or more types of networking interfaces that facilitate wireless communication between one or more components of the remote patient monitoring system 100 and/or between the remote patient monitoring system 100 and one or more external components. In embodiments, the networking unit 420 may operatively connect the remote patient monitoring system 100 to a communications network 435, which can include a direct interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some examples, remote patient monitoring system 100 may communicate with one or more remote / cloud-based servers (e.g., electronic medical records database 440), and other data sources such as cloud-based services, and/or one or more devices (e.g., coach devices 315 and/or patient devices 325) via the networking unit 420. In particular embodiments, the coach devices 315 and/or patient devices 325 may access the corresponding coach interfaces 310 and patient interfaces 320 via the networking unit 420.

The memory 410 of the remote patient monitoring system 100 can be variously embodied in one or more forms of machine-accessible and machine-readable memory. In some examples, the memory 410 may comprise one or more types of memory, including one or more types of transitory and/or non-transitory memory. In particular embodiments, the memory 410 can comprise a magnetic disk storage device, an optical disk storage device, an array of storage devices, a solid-state memory device, and/or the like, including combinations thereof. The memory 410 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like.

In particular embodiments, the memory 410 can be configured to store data 445 and machine-readable instructions 450 that, when executed by the one or more processors 405, cause the remote patient monitoring system 100 to perform one or more steps of the methods and/or processes described herein. For example, as described herein, the instructions 450 may include one or more of: a data collection module configured to obtain patient data for a plurality of patients from one or more data sources; an intervention prioritization module configured to identify patients from among a large population of remotely monitored patients that are most likely to benefit from engagement / intervention as described herein; and an engagement recommendation engine configured to recommend and organize potential intervention actions according to the determined intervention priority scores of the relevant patients.

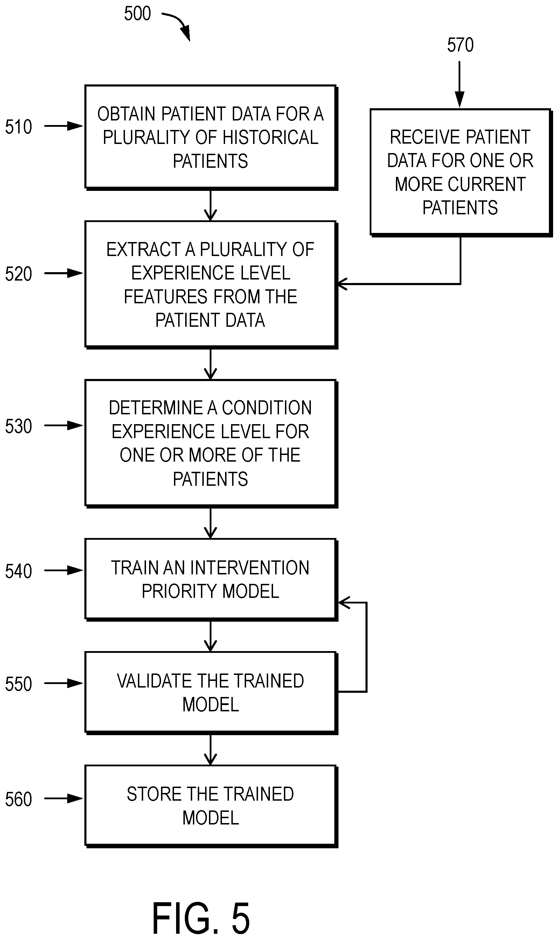

In particular embodiments, the instructions 450 may also include a model training component, which can be a stored program component that is executed by at least one processor, such as the one or more processors 405 of the remote patient monitoring system 100. The model training component can be configured to train one or more intervention priority prediction models 305 using available data. For example, with reference to FIG. 5, a process 500 for training an intervention priority prediction model 305 is illustrated according to aspects of the present disclosure. As shown, the process 500 includes: in a step 510, obtaining historical patient data for a plurality of historical patients; in a step 520, extracting a plurality of experience level features for each of the historical patients; in a step 530, determining a condition experience level for at least some of the historical patients based on the experience level features extracted; in a step 540, training the intervention priority prediction model 305 using the condition experience level determined for the historical patients; optionally, in a step 550, validating the trained intervention priority prediction model 305; and in a step 560, storing the trained intervention priority prediction model 305. In some embodiments, the process 500 may also include, in a step 570, receiving newly generated patient data for one or more current patients and retraining the intervention priority prediction model 305 based on the newly generated patient data.

More specifically, in the step 510, the process 500 includes obtaining a model training dataset, which can include receiving patient data for a plurality of historical patients from one or more data sources. As described above and illustrated in Table 1, the patient data can include demographic information, such as age, gender, sex, race, ethnicity, marital status, and/or the like; patient-reported outcome measures (PROMS), including general and condition-specific PROMS; patient-reported experience measures (PREMS), including general and condition-specific PREMS; system usage data, such as logins, time active, activities performed, devices used, and/or the like; clinical data, such as various at-home test results and/or lab test results; socio-economic data, including education level, zip code, urbanity, and/or the like; and historical intervention action data, including records of past intervention actions and/or educational materials provided.

In embodiments, the model training dataset includes at least three types of information for at least some of the historical patients, including (1) information from which a condition experience level can be determined, (2) actual intervention action data for one or more historical patients, and (3) observations of one or more patient outcomes for one or more historical patients.

At a step 520, the process 500 includes extracting a plurality of experience level features from the training dataset and/or online datasets (i.e., newly-available information for patients not included in the training dataset). In embodiments, these experience level features may be extracted as described above. Similarly, at a step 530, the process 500 includes determining a condition experience level for at least some of the historical patients and/or newly-identified patients as described above.

At a step 540, the process 500 includes training the one or more intervention priority prediction models 305 using the condition experience levels determined for at least the historical patients and the actual intervention action data as sample inputs and the changes in patient outcomes for the one or more historical patients as sample outputs. As described below, the prediction models 305 may be updated periodically updated based on newly-available patient information.

At a step 550, the process 500 optionally includes validating the trained intervention priority prediction model 305. In embodiments, cross-validation may be used for training (in the step 550) and validating. In particular, parameters such as area under the curve (AUC) or area under the receiver operating characteristic (AUROC), accuracy, and mean-squared error can be used to compare the model's performance on a test dataset. Parameters such as weighted accuracy and weighted mean-square error may also be used. As shown in FIG. 5, the training (step 540) and validation (step 550) operations may be repeated a number of times until the desired performance is obtained.

At a step 560, the process 500 includes storing the one or more trained intervention priority prediction models 305. For example, the trained intervention priority prediction models 305 may be stored in the memory 410 of the remote patient monitoring system 100 for later use and/or distribution.

In some embodiments, the process 500 can also include a step 570, wherein the trained intervention priority prediction model 305 is updated based on newly obtained patient data including records of additional intervention actions being taken and any changes in the patient outcomes observed following the implementation of those intervention actions. More specifically, as shown in FIG. 5, updating the trained prediction model 305 can include receiving online datasets (i.e., newly-available information for patients not included in the training dataset), which then undergoes feature extraction and condition experience level analysis as described with respect to steps 520, 530 before being used to retrain the trained prediction model 305.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, procedural programming languages such as the "C" programming language or similar programming languages, declarative programming languages including logic programming languages like SQL and functional programming languages like R, symbolic programming languages like LISP, and/or other programming language paradigms. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A remote patient monitoring system (100) configured to provide engagement guidance in connection with a plurality of patients, the system (100) comprising:
one or more processors (405); and
memory (410) having stored thereon machine-readable instructions (450) that, when executed by the one or more processors (405), cause the one or more processors (405) to perform operations comprising:
(i) obtain patient data for at least a first subset of patients from one or more data sources;
(ii) extract a plurality of experience level features for each patient of at least the first subset of patients from the patient data obtained;
(iii) determine a condition experience level for each patient of at least the first subset of patients based on the experience level features extracted for the corresponding patient;
(iv) generate an intervention priority score for each patient of at least the first subset of patients by applying a trained intervention priority model (305) to the condition experience level determined for the corresponding patient, wherein each intervention priority score is indicative of a likelihood of the corresponding patient having a positive change in one or more clinical outcomes following one or more intervention actions; and
(v) automatically generate a recommended intervention plan for each patient of at least the first subset of patients based on the intervention priority scores generated for at least the first subset of patients, wherein each recommended intervention plan includes at least one intervention action.

2. The remote patient monitoring system (100) of claim 1, wherein the intervention priority model (305) is trained by:
(i) obtaining historical patient data for a plurality of historical patients, wherein the historical patient data includes historical intervention action data and actual patient outcome data for the plurality of historical patients;
(ii) extracting a plurality of experience level features for each historical patient from the historical patient data obtained;
(iii) determine a condition experience level for each historical patient based on the experience level features extracted for the corresponding historical patient;
(iv) training the intervention priority model using the condition experience levels determined for each of the historical patients and the historical intervention action data for each of the historical patients as inputs and changes in actual patient outcome data for each of the historical patients as outputs; and
(v) storing the trained intervention priority model (305).

3. The remote patient monitoring system (100) of claim 1, further comprising:
one or more patient interfaces (320) configured to be executed by or otherwise accessible via one or more patient devices (325), each patient interface (320) being configured to send and receive data related to the remote monitoring of a corresponding patient (335); and
one or more coach interfaces (310) configured to be executed by or otherwise accessible via one or more coach devices (315), each coach interface (310) being configured to send and receive data related to the remote monitoring of a plurality of patients (335).

4. The remote patient monitoring system (100) of claim 1, wherein each recommended intervention plan includes at least one intervention action identified based on the intervention priority score generated for the corresponding patient.

5. The remote patient monitoring system (100) of claim 1, wherein the patient data comprises one or more of the following: demographic information; patient-reported outcome measures; patient-reported experience measures; user interface usage data; system usage data; clinical data; socio-economic data; and historical intervention action data.

6. The remote patient monitoring system (100) of claim 1, wherein the intervention priority score generated for each patient includes a plurality of sub-scores corresponding to one or a combination of potential intervention actions, each sub-score being indicative of a likelihood of the corresponding patient having a positive change in one or more clinical outcomes following the one or the combination of potential intervention actions.

7. The remote patient monitoring system (100) of claim 1, wherein the condition experience level determined for each patient has a first component comprising a score based on one or more current clinical values associated with the corresponding patient, and a second component comprising a score based on evidence of one or more historical intervention actions.

8. A non-transitory computer-readable storage medium (410) having stored thereon machine-readable instructions (450) that, when executed by one or more processors (405), cause the one or more processors (405) to perform operations comprising:
(i) obtaining patient data for at least a first subset of patients from one or more data sources;
(ii) extracting a plurality of experience level features for each patient of at least the first subset of patients from the patient data obtained;
(iii) determining a condition experience level for each patient of at least the first subset of patients based on the experience level features extracted for the corresponding patient;
(iv) generating an intervention priority score for each patient of at least the first subset of patients by applying a trained intervention priority model (305) to the condition experience level determined for the corresponding patient, wherein each intervention priority score is indicative of a likelihood of the corresponding patient having a positive change in one or more clinical outcomes following one or more intervention actions; and
(v) automatically generating a recommended intervention plan for each patient of at least the first subset of patients based on the intervention priority scores generated for at least the first subset of patients, wherein each recommended intervention plan includes at least one intervention action.

9. The non-transitory computer-readable storage medium (410) of claim 8, wherein the intervention priority model (305) is trained by:
(i) obtaining historical patient data for a plurality of historical patients, wherein the historical patient data includes historical intervention action data and actual patient outcome data for the plurality of historical patients;
(ii) extracting a plurality of experience level features for each historical patient from the historical patient data obtained;
(iii) determine a condition experience level for each historical patient based on the experience level features extracted for the corresponding historical patient;
(iv) training the intervention priority model using the condition experience levels determined for each of the historical patients and the historical intervention action data for each of the historical patients as inputs and changes in actual patient outcome data for each of the historical patients as outputs; and
(v) storing the trained intervention priority model (305).

10. A computer-implemented method (200) of providing engagement guidance in connection with a plurality of patients using a remote patient monitoring system (100), the method (200) comprising:
obtaining patient data for at least a first subset of patients from one or more data sources;
extracting a plurality of experience level features for each patient of at least the first subset of patients from the patient data obtained;
determining a condition experience level for each patient of at least the first subset of patients based on the experience level features extracted for the corresponding patient;
generating an intervention priority score for each patient of at least the first subset of patients by applying a trained intervention priority model (305) to the condition experience level determined for the corresponding patient, wherein each intervention priority score is indicative of a likelihood of the corresponding patient having a positive change in one or more clinical outcomes following one or more intervention actions; and
automatically generating a recommended intervention plan for each patient of at least the first subset of patients based on the intervention priority scores generated for at least the first subset of patients, wherein each recommended intervention plan includes at least one intervention action.

11. The computer-implemented method (200) of claim 10, wherein the intervention priority model (305) is trained by:
(i) obtaining historical patient data for a plurality of historical patients, wherein the historical patient data includes historical intervention action data and actual patient outcome data for the plurality of historical patients;
(ii) extracting a plurality of experience level features for each historical patient from the historical patient data obtained;
(iii) determine a condition experience level for each historical patient based on the experience level features extracted for the corresponding historical patient;
(iv) training the intervention priority model using the condition experience levels determined for each of the historical patients and the historical intervention action data for each of the historical patients as inputs and changes in actual patient outcome data for each of the historical patients as outputs; and
(v) storing the trained intervention priority model (305).

12. The computer-implemented method (200) of claim 10, further comprising:
presenting, via one or more coach interfaces of the remote patient monitoring system, the recommended intervention plans for at least some of the first subset of patients;
receiving, via the one or more coach interfaces of the remote patient monitoring system, a selection of one or more intervention actions from the recommended intervention plans of at least one patient; and
automatically implementing, via one or more patient interfaces, at least one intervention action of the one or more selected intervention actions, wherein the at least one intervention action is implemented in connection with at least a first patient.

13. The computer-implemented method (200) of claim 12, further comprising:
determining a change in one or more patient outcomes for at least the first patient following the implementation of at least the one intervention action; and
updating the trained intervention priority model (305) using an updated training dataset that includes the at least one intervention action and the change in one or more patient outcomes for at least the first patient.

14. The computer-implemented method (200) of claim 10, wherein generating an intervention priority score for each patient of at least the first subset of patients includes generating a plurality of sub-scores for each patient of at least the first subset of patients by applying the trained intervention priority model (305), wherein each sub-score is indicative of a likelihood of the corresponding patient having a positive change in one or more clinical outcomes following one or combination of potential intervention actions.

15. The computer-implemented method (200) of claim 10, the patient data comprises one or more of the following: demographic information; patient-reported outcome measures; patient-reported experience measures; user interface usage data; system usage data; clinical data; socio-economic data; and historical intervention action data.
